# EUROPEAN PATENT APPLICATION

(11) **EP 4 316 548 A1**
(43) Date of publication of application: **07.02.2024**
(21) Application number: 23188983.3
(22) Date of filing: 01.08.2023
(51) Int. Cl.: A61M 5/142, A61B 5/00, A61F 13/02

(54) **THERMAL WELD STRUCTURES FOR REDUCING TEARING OF AN ADHESIVE LAYER FOR AN ON-BODY MEDICAL DEVICE**

(30) Priority: 01.08.2022 US 202263370011 P
(71) Applicant: Insulet Corporation, Acton MA 01720 (US)
(72) Inventor: BARNES, Jeffrey, Medford, 02115 (US)
(74) Representative: Peterreins Schley

(57) **Abstract**

The exemplary embodiments provide thermal weld structures that help prevent tearing of the adhesive layer of an on-body medical device when subject to lateral forces. These thermal weld structures help reduce the tearing by providing sacrificial thermal weld structures that will absorb forces and then potentially fail to thereby diffuse some of the lateral forces. The sacrificial thermal weld structures may take different forms. For instance, the sacrificial thermal weld structures may be gradient thermal weld structures where the amount of material melted in the gradient thermal weld structures decreases as a gradient along a dimension of the structures, such as their length. In some alternative embodiments, the width of the gradient thermal weld structure may vary instead of the height, or in conjunction with the height. In other exemplary embodiments, the sacrificial thermal weld structures may be dot thermal weld structures.

## Description

### BACKGROUND

Many on-body medical devices, such as insulin patch pumps, are secured to users by using adhesive layers. The adhesive layers include adhesives that are designed to stick to the skin of the users on arms, legs, backs or torso or other skin surfaces to hold the on-body medical devices securely to the users. Each adhesive layer typically has a sheet of a substrate material upon which there is a coating of adhesive. The adhesive layer is secured to a surface of the on-body medical device, such as an exterior surface of a housing. Thermal welds formed by melting a meltable material, like a plastic, may be used to secure the adhesive layer to the surface.

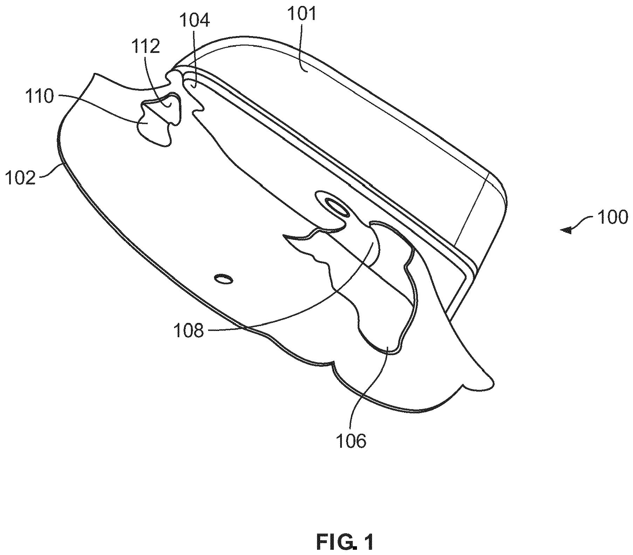

One difficulty encountered with some conventional on-body medical devices is that the adhesive layers of the conventional on-body medical devices have a tendency to tear when subject to lateral forces, such as when the on-body medical devices are accidently struck by users or the conventional on-body medical devices strike objects, like walls, furniture, clothing, etc. Figure 1 shows an example of a conventional on-body medical device 100 with an adhesive layer 102 that was secured to a surface 104 of a housing 101 with a tear 106 near thermal plastic weld 108. In addition, there is a tear 110 near thermal plastic weld 112. The thermal plastic welds 108 and 112 are substantial welds that are unlikely to fail under typical use conditions. The lateral forces cause the tears near the welds 108 and 112.

### SUMMARY

In accordance with a first inventive facet, an on-body medical device includes a surface, such as a skin-facing bottom exterior surface of a housing, and an adhesive layer for securing the medical device to the skin of a user. The adhesive layer contains a substrate on which an adhesive is applied. The on-body medical device further includes meltable weld features formed on the surface for securing the adhesive layer to the surface when melted. The meltable thermal weld features include a first meltable structure of a substantially uniform amount of a first meltable material above a melting border along a length of the first structure. A portion of the first meltable material at a height above the melting border is configured to melt. The meltable thermal weld features also include a second meltable structure, wherein the second meltable structure is configured to have a gradient in amount of a second meltable material above a second melting border that extends along at least a portion of a length of the second meltable structure that is configured to melt.

The first meltable structure may be of a substantially uniform height above the melting border. The second meltable structure may include a gradient structure of the second meltable material that decreases in height above the second melting border along at least a portion of the gradient structure. The on-body medical device additionally may include an additional gradient structure of the second meltable material that decreases in height above a third melting border along at least a portion of the additional gradient structure. The surface may have a width, such that the first meltable structure extends across a central portion of the width of the surface, and the second meltable structure extends across a portion of the surface between the central portion of the width and an edge of the surface. The on-body medical device may further include an additional meltable structure configured to have a gradient in amount of meltable material above a third melting border along at least a portion of its length. The second meltable structure and the additional meltable structure may be formed on opposite ends of the first meltable structure along the width of the surface. The second meltable structure and the additional meltable structure may be arcuate raised ribs of the meltable material. The first structure may comprise a raised rib of the meltable material. The first meltable material may be the same meltable material as the second meltable material.

In accordance with another inventive facet, an on-body medical device includes a surface and an adhesive layer for securing the medical device to skin of a user. The adhesive layer contains a substrate on which an adhesive is applied. The device also includes meltable thermal weld features formed on the surface for securing the adhesive layer to the surface when melted. The meltable thermal weld features include a first meltable structure of a first meltable material having a substantially uniform amount of the first meltable material above a melting border along a length of the first meltable structure. The first meltable material is at a height above the melting border and is configured to melt. The meltable thermal weld features further include a second meltable structure of a second meltable material. The second meltable structure is configured to have a gradient in amount of the second meltable material above a second melting border that extends along at least a portion of a length of the second meltable structure. The meltable thermal weld features additionally include at least one spot weld feature of a third meltable material formed on the surface positioned and configured to be weakest of the weld features once melted to form a thermal weld.

The at least one spot weld feature may include multiple spot weld features. The at least one spot weld feature may be a single spot weld feature positioned between the second meltable feature and an edge of the surface. The first meltable material, the second meltable material and the third meltable material may be the same meltable material or may be different meltable materials.

In accordance with an additional inventive facet, an on-body medical device includes a surface and an adhesive layer for securing the medical device to skin of a user. The adhesive layer contains a substrate on which an adhesive is applied. The device also includes a main thermal weld structure containing meltable material for securing the adhesive layer to the surface and dot thermal weld structures containing the meltable material for additional securing of the adhesive layer to the surface.

The dot thermal weld structures may include dot thermal weld structures of different diameters. The dot thermal weld structures may include dot thermal weld structures of different heights. The dot thermal weld structures may be grouped into spatially segregated groups on the housing. At least one of the spatially segregated groups may contain thermal weld structures of different heights. At least one of the spatially segregated groups may contain thermal weld structures of different diameters. Average spacing between dot thermal weld structures of a first of the spatially segregated groups is at least 20 percent less than the average spacing between dot weld thermal structures of a second of the spatially segregated group. The dot thermal weld structures may be positioned adjacent or around critical locations such as an infusion site.

### BRIEF DESCRIPTION OF THE DRAWINGS

Figure 1 depicts an illustrative of an adhesive layer of a conventional on-body medical device tearing.
Figure 2A depicts an example of thermal weld patterns for a conventional on-body medical device.
Figure 2B depicts an on-body medical device that is suitable for exemplary embodiments.
Figure 3 depicts a flowchart of illustrative steps that may be performed in exemplary embodiments to create a thermal weld.
Figure 4 depicts a flowchart of illustrative steps that may be performed in exemplary embodiments to create thermal weld structures of varying strengths.
Figures 5A, 5B and 5C depict an illustrative configuration of an exemplary embodiment where two varieties of strengths of thermal weld structures are formed.
Figures 6A, 6B and 6C illustrate a melting border and what portions of the thermal weld structures are configured to melt during thermal welding.
Figure 7 depicts a flowchart of illustrative steps that may be performed in exemplary embodiments to add dot thermal welds to an on-body medical device.
Figure 8 depicts an example of an exemplary embodiment that includes a dot thermal weld structure, a gradient weld and a large uniform thermal weld.
Figure 9 depicts a melting boundary for the arrangement of Figure 8 in an exemplary embodiment.
Figure 10 depicts a flowchart of illustrative steps that may be performed in exemplary embodiments to deploy dot thermal weld structures with a main thermal weld structure.
Figure 11 depicts an illustrative on-body medical device of exemplary embodiments having dot thermal weld structures and a main thermal weld structure.

### DETAILED DESCRIPTION

The exemplary embodiments provide thermal weld structures that help prevent tearing of the adhesive layer of an on-body medical device when subject to forces. These thermal weld structures help reduce the tearing by providing sacrificial thermal weld structures that will absorb forces and then potentially fail to thereby diffuse some of the lateral forces. In particular, the sacrificial thermal weld structures may break during a sudden impact which may result in a spike in lateral forces. The sacrificial thermal weld structures may diffuse or dissipate some of the lateral forces of the spike, thereby protecting the first meltable structure from breaking under the sudden load and hence keeping the on-body medical device securely attached to the adhesive layer and thereby the user. Merely increasing the amount of thermal weld structure between the adhesive layer and the surface of the on-body medical device may result in discomfort to the user as the adhesive layer cannot conform to the user's skin due to the on-body medical device acting as a reinforcement to the adhesive layer. Additionally, simply increasing the amount of thermal weld structure may result in the on-body medical device being unable to tilt relative to the skin without part of the total the thermal weld structure breaking. The on-body medical device tilting to a certain degree may occur for example when a patient is putting on clothes. The sacrificial thermal weld structures may take different forms. For instance, the sacrificial thermal weld structures may be gradient thermal weld structures where the amount of material melted in the gradient thermal weld structures decreases as a gradient along a dimension of the structures, such as their length. In some exemplary embodiments, the height of such a gradient thermal weld structure decreases along its length. There is less material that is melted as the height of the gradient thermal weld structure decreases assuming a constant width along the length, and as a result, the welds become less strong as the height decreases. In some alternative embodiments, the width of the gradient thermal weld structure may vary instead of the height or in conjunction with the height. In some embodiments, the height of the gradient thermal weld structure at its smallest point is between about 10% to about 99%, more specifically between about 50% to about 90% and in particular between about 70% to about 85% smaller than the gradient thermal weld structure at its highest points. In some embodiments, the width of the gradient thermal weld structure at its thinnest point is between about 10% to about 99%, more specifically between about 50% to about 90% and in particular between about 70% to about 85% thinner than the gradient thermal weld structure at its highest points.

In other exemplary embodiments, the sacrificial thermal weld structures may be dot thermal weld structures. The dot thermal weld structures may each be a discrete thermal weld structure with a profile resembling a circular, square or rectangular dot. These dot thermal weld structures may be positioned to be the first thermal weld structures to fail to dissipate lateral forces. This helps to reduce tears in the adhesive layer and to avoid failure of more substantial thermal weld structures. In some embodiments, the dot thermal weld structures may have a maximum diameter, wherein the maximum diameter is between about 0.5 mm to about 10 mm, more specifically between about 1 mm to about 7 mm and in particular between about 2 mm to about 5 mm. In some embodiments, the dot thermal weld structures may have a minimum diameter, wherein the minimum diameter is between about 0.05 mm to about 5 mm, more specifically between about 0.2 mm to about 3 mm and in particular between about 0.5 mm to about 2 mm.

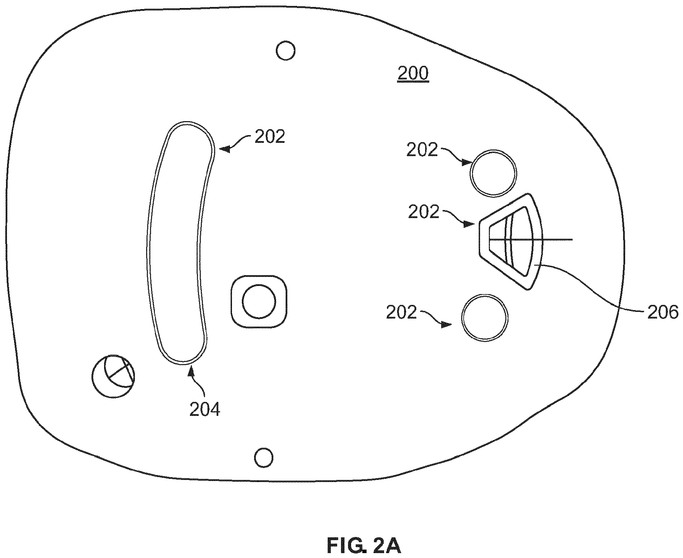

Figure 2A depicts illustrative weld patterns on an adhesive layer 200 for a conventional on-body medical device. The depicted device is an insulin pump for delivering insulin to a user. The weld lines 202 form a "smile" weld feature 204. This feature 204 is the primary thermal weld for holding the adhesive layer 200 secure to the bottom surface of the housing of the insulin pump. As can be seen, this feature 204 spans a substantial portion of the width of the adhesive layer and the underlying surface of the housing to which it is attached. The other weld lines 202 secure the area around a needle/cannula assembly. Since it is important for the cannula to stay in the user after insertion, the area around the infusion site or the needle/cannula assembly insertion area is reinforced by the thermal welds to ensure the adhesive layer does not separate or tear which could result in the cannula pulling out of the user.

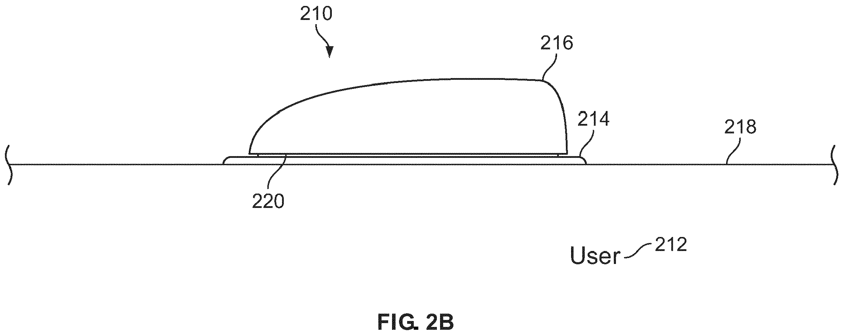

Figure 2B depicts a side view of an on-body medical device 210 of exemplary embodiments. The on-body medical device may, in some embodiments, be a medicament delivery device for delivering medicament to a user 212, such as an insulin pump for delivering insulin to the user 212. The on-body medical device includes a housing 216 for housing electrical and mechanical components, such as a medicament reservoir, a medicament pump and a processor for running a control system for the on-body medical device. An adhesive layer 214 secures the on-body medical device to the skin 218 of the user 212. The adhesive layer has a substrate, such as a layer of a polyester weave, to which a medical adhesive is applied. The substrate layer is secured to a surface of the on-body medical device, such as a bottom exterior surface of the housing 216. In the exemplary embodiments, the adhesive layer is thermally welded to the surface of the on-body medical device.

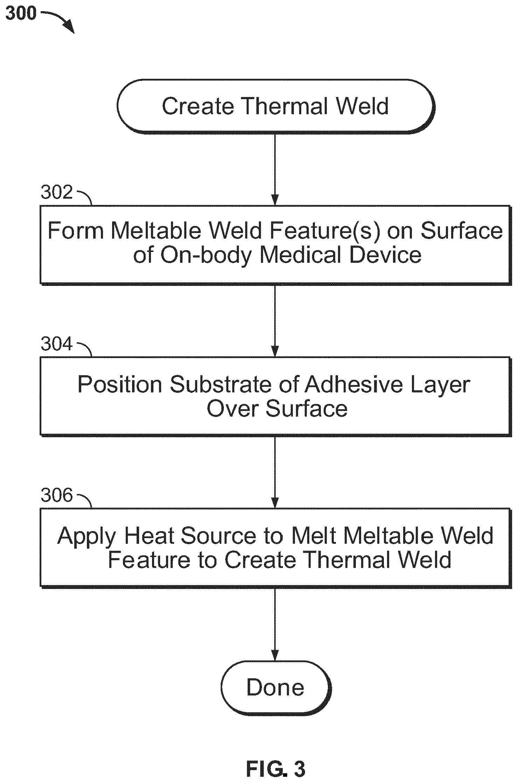

Figure 3 depicts a flowchart 300 of illustrative steps that may be performed in exemplary embodiments to create welds using the meltable weld features described in more detail below. At 302, meltable weld feature(s) are formed on a surface 220 of an on-body medical device. For instance, the meltable weld feature(s) may be formed on a bottom exterior surface 220 of the housing 216 for the on-body medical device 210. Alternatively, the meltable weld feature may be formed on a stand or other surface that is formed to provide an interface with the skin 218 surface of the user 212. At 304, the substrate of the adhesive layer 214 may be positioned to cover the surface to which the adhesive layer is to be thermal welded. At 306, a heat source is then applied to melt the meltable weld feature to secure the adhesive layer to the surface 220.

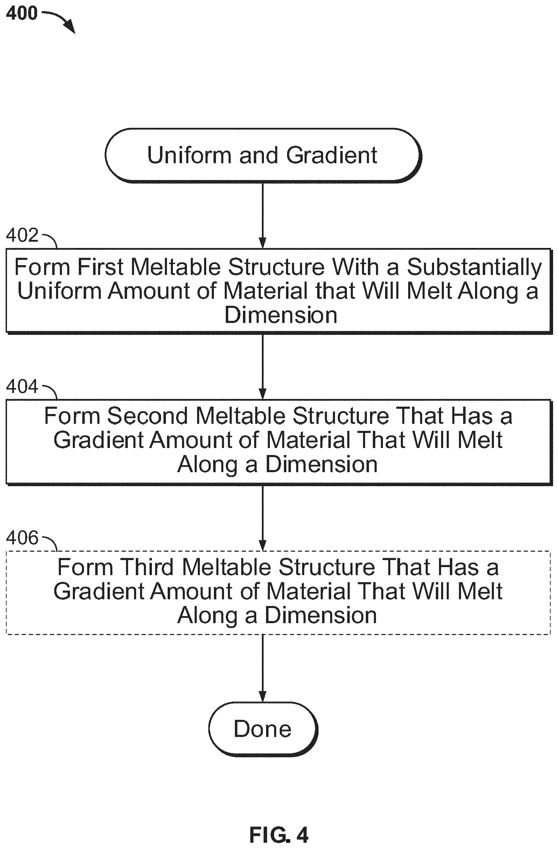

Figure 4 depicts a flowchart 400 of illustrative steps that may be performed in exemplary embodiments to form meltable weld features with gradient weld features. At 402, a first meltable structure is formed. The first meltable weld structure has a substantially uniform amount of material that will melt along a dimension, such as length or width. The first meltable weld structure may be made of a first meltable material, such as a plastic. At 404, a second meltable structure that has a gradient amount of material that will melt along a dimension, such as length or width. The gradient structure will produce weld locations that get progressively weaker or stronger along the dimension due to the gradient of meltable material. The gradient may be positioned so that weakest weld point with the least melted material may be located near the edge of the surface and/or the edge of the surface. Accordingly, in some embodiments, the weakest welding or the weld point with the least melted material may be located closest to the edge of the surface compared to any other weld point. Thus, lateral forces will affect the weakest weld point, which may break along a gradient to dissipate the lateral forces. The weld points may get progressively stronger along the length heading toward the interior of the adhesive surface so that the weaker welds are positioned near the edge of the surface and adhesive layer to fail in succession to dissipate the lateral forces to save the stronger welds or welds in more critical locations such as around the infusion site.

In some exemplary embodiments, at 406, an optional third meltable structure may be formed. Like the second meltable structure, the third meltable structure may have a gradient amount of material that will melt along a dimension. It should be appreciated that additional meltable structures with gradients of meltable material or a uniform distribution of meltable material may be formed on the surface in some exemplary embodiments.

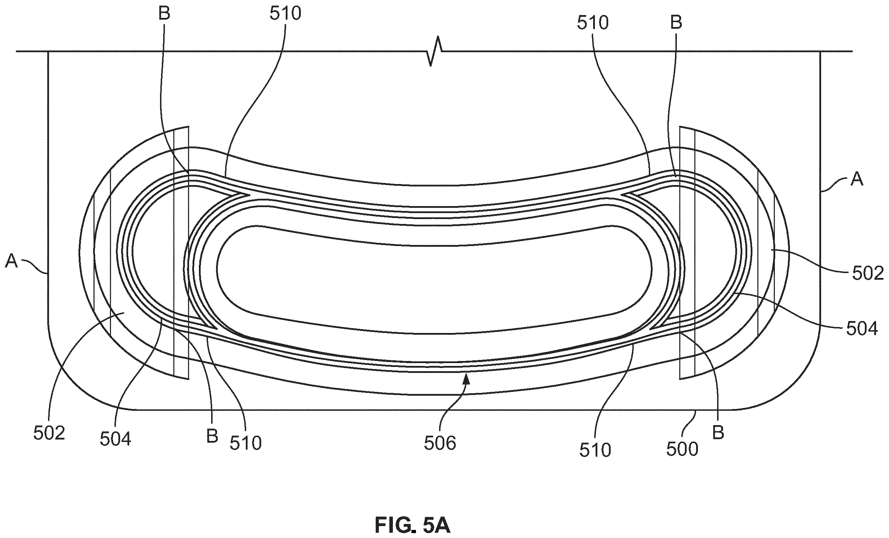

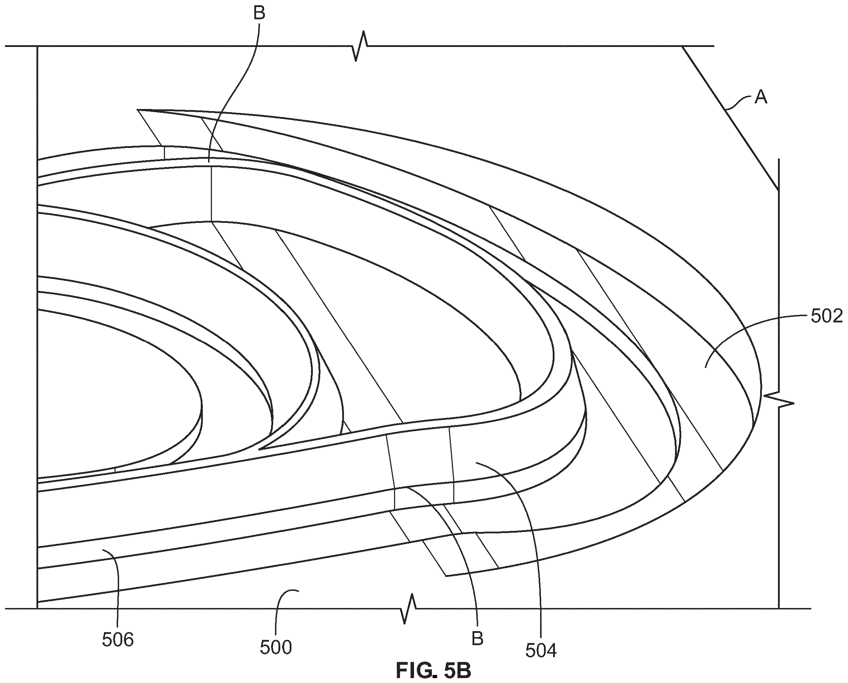

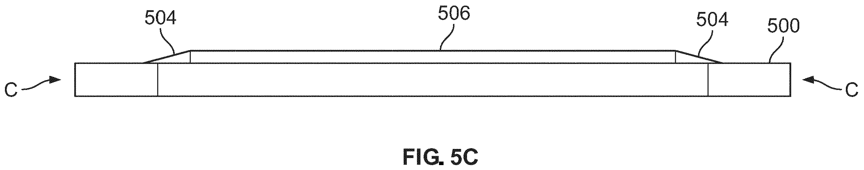

Figures 5A, 5B and 5C depict one arrangement of illustrative meltable weld features of an exemplary embodiment. A first meltable structure 506 is formed on a surface, such as the bottom exterior surface 500 of a housing of an on-body medical device, like an insulin pump or other type of medicament delivery device. The first meltable structure 506 may have a uniform height and width along its length. The first meltable structure 506 may be formed of a meltable plastic or other suitable material. In the illustrative example shown, the first meltable structure 506 has a largely oval portion 508 and extending arms 510 that join with arcuate second and third meltable structures 504. The second and third meltable structures 504 are formed of second and third meltable materials, respectively and do not have a uniform amount of meltable material along the length of their arcs due to a taper or a change in height of the top surface. Instead, the second and third meltable structures have a gradient of meltable material configured to melt that gradually decreases as the arcuate members extend from the junction points B toward their respective edge A. In some embodiments, the first, second and/or third meltable material may have a melting point between about 60 °C to about 327 °C, more specifically between about 100 °C to about 280 °C and in particular between about 130 °C to about 230 °C.

The gradient is created by the decreasing height of the arcuate structures 504 being in wells 502 that are designed for collecting the melted material during creation of the thermal welds. This can be seen more clearly in Figures 5B and 5C. As can be seen, the well slopes downward as it progresses toward edge A. The raised rib that forms the second meltable structure 504 is formed on the downward facing slope of the well 502. As a result, the height of the second and third meltable structures relative to the surface 500 of the housing decreases toward the edge A. This means that there will be a gradient of meltable material for the second and third meltable structures 504. In general, only material situated above the surface of the plane of surface 500 will potentially melt.

The meltable materials of the first structure 506, the second and third structures 504 may all differ or may all be the same. Still further, some may be the same material with the other being a different meltable material.

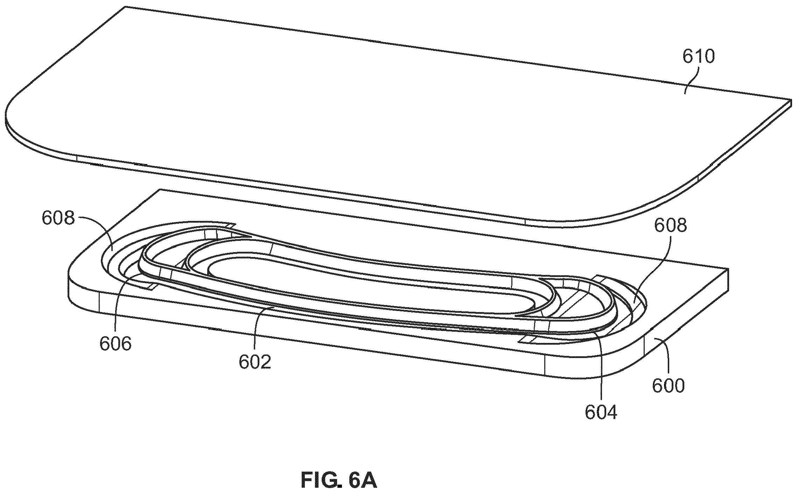

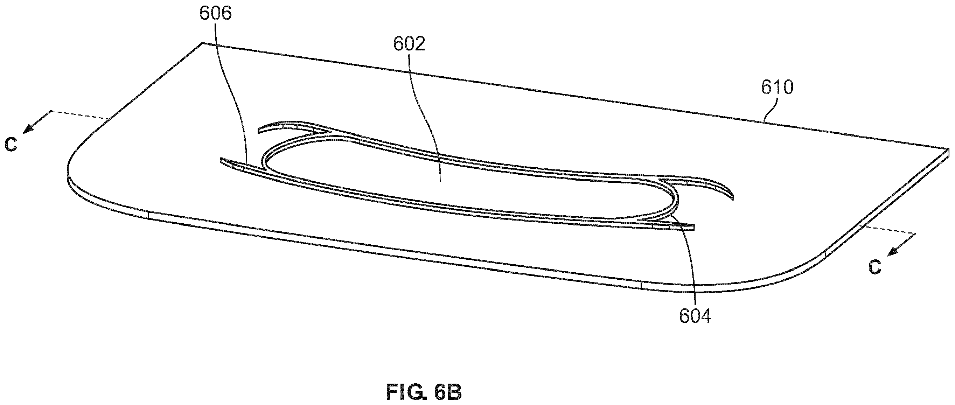

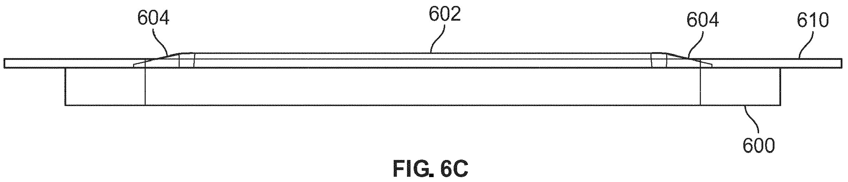

The plane of the surface where there are no meltable features forms a melting boundary that delineates the portion of the meltable material that is configured to melt from the portion of the material that is configured to not melt. Figure 6A depicts a portion of the surface of the housing 600 containing the first meltable structure 602, the second meltable structure 604, the third meltable structure 606, wells 608 and an adhesive layer 610 in a partially exploded view. Figure 6B depicts the interference of first meltable structure 602, the second meltable structure 604 and the third meltable structure 606. The portions of these meltable structures 602, 604 and 606 shown in Figure 6B represent the portion of the structures that are configured to melt when the thermal weld is formed. Thus, the top surface of the adhesive layer defines a melting layer boundary (see the difference in height of the adhesive layer and the meltable structures in Figure 6C). As can be seen, the height for the interfering portion of the first structure 602 is uniform throughout. In contrast, the height of the interfering portion of the second meltable structure 604 and the height of the interfering portion of the third meltable structure 606 decrease as they extend toward the respective edges C of the adhesive layer 610. The net result is that the thermal welds for the second meltable structure and the third meltable structure gets weaker due to the decreasing amounts of meltable material for the welds as they extend toward the edges C, or in other words, a decrease in the amount of material that is melted moving toward the edges C.

In some exemplary embodiments, dot thermal weld structures may be used. The dot thermal weld structures are discrete elements of small diameters of meltable material. The dot welds may be melted to form small thermal welds at points to secure the adhesive layer 606. The dot welds may be useful in providing sacrificial thermal welds that will break at lower lateral forces than more substantial thermal welds. The cross-sectional profile of the dot weld structures may be circular, square, rectangular, oval, or the like.

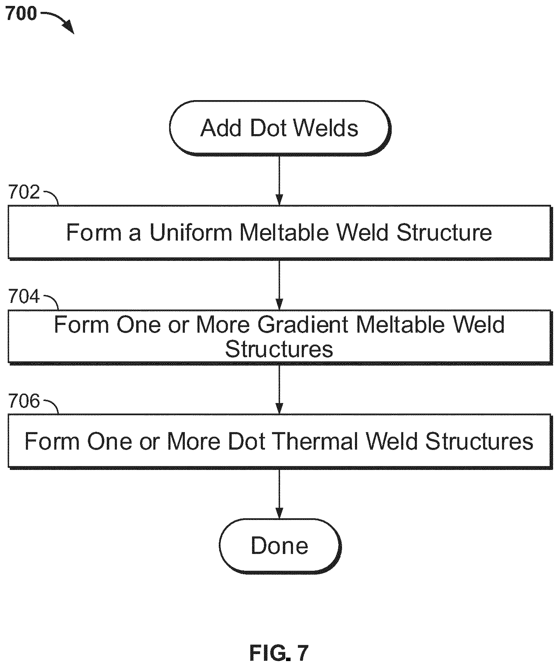

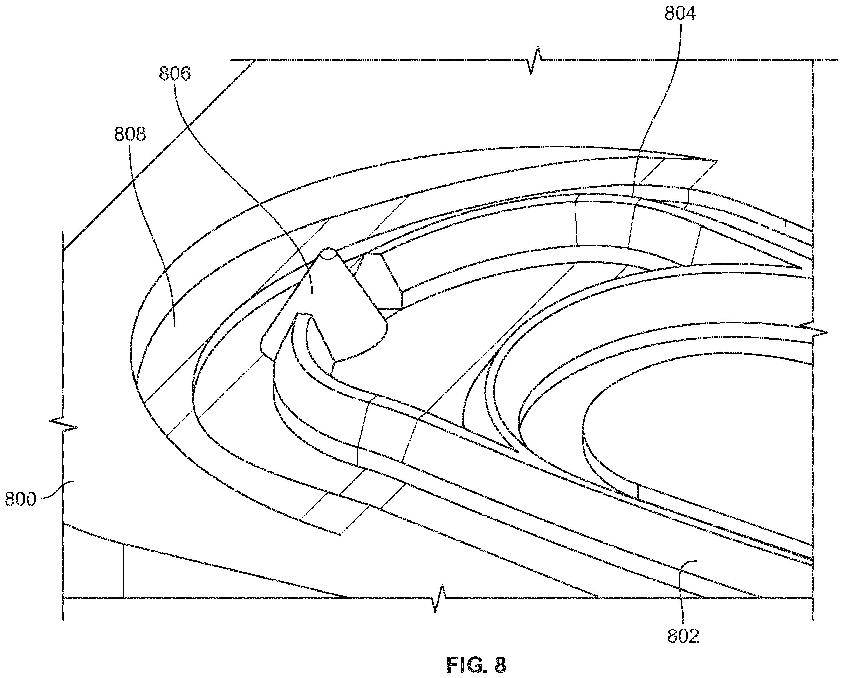

Figure 7 depicts a flowchart 700 of illustrative steps that may be performed in exemplary embodiments relating to a configuration of three layers of thermal welds of differing strengths, including dot thermal welds. In these exemplary embodiments, at 702, a uniform meltable thermal weld structure 802 may be provided on the surface of the housing 800 or other skin facing surface of the on-body medical device to which the adhesive layer is to be attached. For instance, a uniform meltable thermal weld structure with a configuration like 506 shown in Figure 5B may be formed on a surface of the housing of the medicament delivery device. At 704, one or more gradient meltable thermal weld structures 804 may be formed on the surface of the housing 800. The gradient meltable thermal weld structures 804, may be modified as shown in Figure 8 relative to the previously described gradient meltable thermal weld structures 504 to have a break in which a dot thermal weld structure of meltable material 806 is formed at 706. The depicted dot thermal weld structure 806 is conical, but the dot thermal weld structure may have other geometric forms, such as spherical, cylindrical, or button-like shapes.

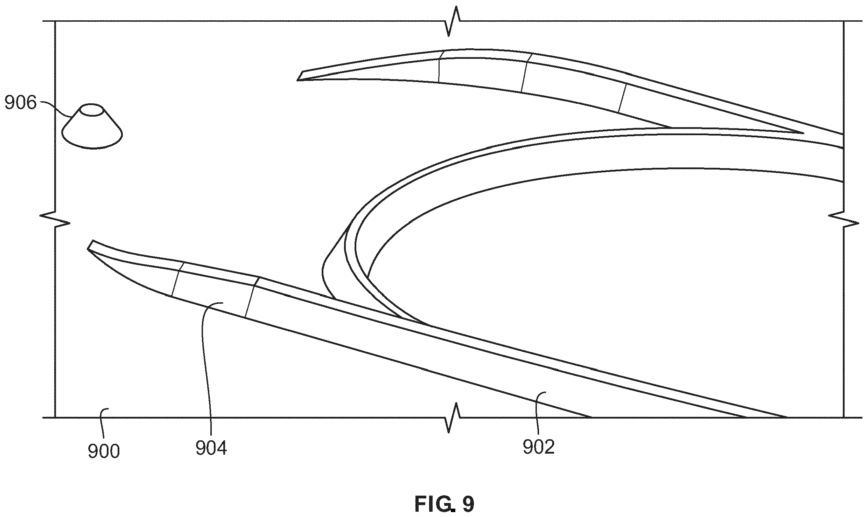

As shown in Figure 9, this configuration provides a three-layer thermal weld structure that provides defense against increasing levels of lateral forces to reduce or prevent tears in the adhesive layer. Figure 9 depicts the interference between the adhesive layer 900 and the thermal weld structures 902, 904 and 906. The adhesive layer 900 represents a melting border. The meltable material amounts above the adhesive layer 900 shown in Figure 9 for the thermal weld structures 902, 904 and 906 are configured to melt and form thermal welds. These depicted amounts correlate with the strengths of the resulting thermal welds. The dot thermal weld structure 906 has a lowest strength among the depicted thermal weld structures and will fail first in dispersing lateral forces. The gradient thermal weld 904 has a next lowest strength and will initially fail at the point on the gradient having the least amount of meltable material. The uniform thermal weld structure 902 has the greatest strength and is designed to not fail during customary use.

In alternative exemplary embodiments, multiple dot thermal weld structures may be used, and the dot thermal weld structures may be positioned in different ways. Further, the amount of meltable material in dot weld structures may vary.

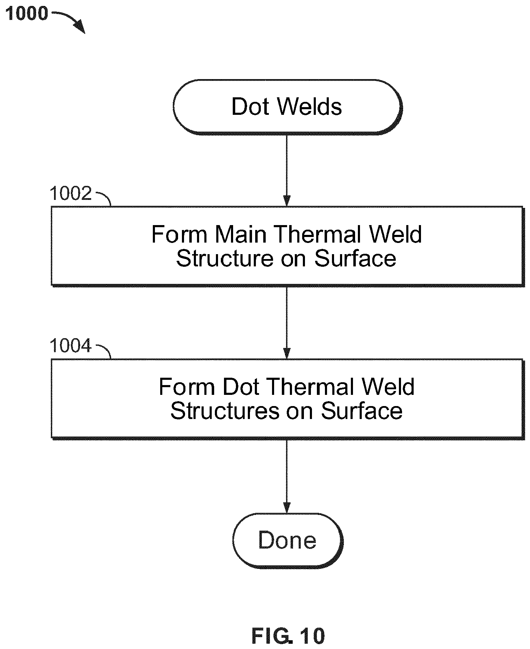

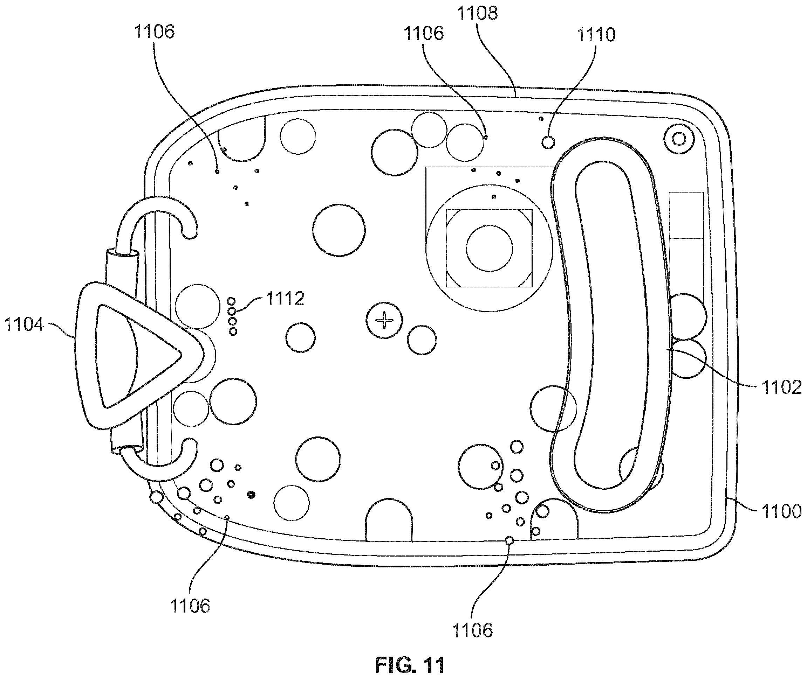

Figure 10 depicts a flowchart of illustrative steps that may be performed in such alternative exemplary embodiments. At 1002, a main thermal weld structure is formed on a surface, such as on a skin facing surface on the exterior of the housing of the on-body medical device. Figure 11 depicts an example of an on-body medical device with a thermal weld 1102 formed by such a thermal weld structure on the skin-facing bottom surface 1100 of the housing. At 1002, dot thermal weld structures are formed on the surface of the on-body medical device. Figure 11 depicts four groups 116 of dot thermal weld structures. Each group 116 includes larger dot thermal weld structures 1110 and smaller thermal diameter weld structures 1108 of smaller diameters. The heights of the dot thermal weld structures may also vary. For instance, dot thermal weld structures 1108 may be less than dot thermal weld structures 1110. Two of the groups 1106 are positioned adjacent to the thermal weld 1104 and two are positioned adjacent the main weld 1102. In the configuration shown the smaller thermal weld structures 1108 are positioned closer to the edges along the width of the surface 1100 relative to the larger dot thermal weld structures 1110. The smaller dot thermal weld structures 1108 are weaker and will fail first responsive to sufficient lateral forces. The depicted configuration is intended to be merely illustrative and not limiting. Other configurations may be used. A final group 1112 may have more closely spaced dot thermal weld structures than the other groups 1106. The average spacing between the dot thermal weld structures in group 1112 is more than 20% closer than the other groups 1110. In some embodiments, the average spacing between the dot thermal weld structures in a first group of thermal weld structures is between about 20% to about 90%, more specifically between about 30% to about 80% and in particular between about 40% to about 70% closer (or smaller) than in a second group of thermal weld structures. Thermal weld structures may be placed closer together to better protect more critical areas at the bottom surface, such as around the infusion location of the medicament delivery device. In some embodiments, an area of the medical device around a cannula/needle assembly may comprise a plurality of dot thermal weld structures. In some embodiments, an area of the medicament delivery device around a cannula/needle assembly may comprise the first group of thermal weld structures and a second area of the medicament delivery device comprises the second group of thermal weld structures. The area around the cannula/needle assembly may relate to an area, e.g. a 0.2 cm to 1.5 cm radius, around the cannula/needle when the cannula/needle is in contact with (inserted into) the patient.

In accordance with another inventive facet, an on-body medical device, comprises a surface and an adhesive layer for securing the medical device to skin of a user, the adhesive layer containing a substrate on which an adhesive is applied. The on-body medical device further comprises thermal welds formed on the surface for securing the adhesive layer to the surface. The thermal welds comprise a first weld structure of a substantially uniform amount of a first meltable material along a length of the first structure, and a second weld structure, where the second weld structure has a gradient in amount of a second meltable material that extends along at least a portion of a length of the second weld structure.

In some embodiments, the first weld structure is of a substantially uniform thickness above the melting border.

In some embodiments, the second weld structure comprises a gradient structure of the second meltable material that decreases in thickness along at least a portion of the gradient structure. In some embodiments, the width of the gradient thermal weld structure at its smallest point is between about 10% to about 99%, more specifically between about 50% to about 90% and in particular between about 70% to about 85% smaller than the gradient thermal weld structure at its widest points.

In some embodiments, the body medical device further comprises an additional gradient weld structure of the second meltable material that decreases in thickness along at least a portion of the additional gradient structure.

In some embodiments, the surface has a width, wherein the first weld structure extends across a central portion of the width of the surface, and wherein the second weld structure extends across a portion of the surface between the central portion of the width and an edge of the surface.

In some embodiments, the on-body medical device further comprises an additional weld structure having a gradient in amount of meltable material along at least a portion of its length and wherein the second weld structure and the additional weld structure are formed on opposite ends of the first weld structure along the width of the surface.

In some embodiments, the second weld structure and the additional weld structure are arcuate raised ribs of the meltable material.

In some embodiments, the first weld structure comprises a raised rib of the meltable material.

In some embodiments, the first meltable material is a same meltable material as the second meltable material.

In accordance with another inventive facet, an on-body medical device, comprises a surface and an adhesive layer for securing the medical device to skin of a user, the adhesive layer containing a substrate on which an adhesive is applied. Further, the on-body medical device comprises thermal welds formed on the surface for securing the adhesive layer to the surface. The thermal welds comprise a first weld structure of a first meltable material having a substantially uniform amount of the first meltable material above along a length of the first meltable structure, a second weld structure of a second meltable material, wherein the second meltable structure is configured to have a gradient in amount of the second meltable material above that extends along at least a portion of a length of the second meltable structure, and at least one spot weld feature of a third meltable material formed on the surface positioned and configured to be weakest of the thermal welds.

In some embodiments, the at least one spot weld feature comprises multiple spot weld features.

In some embodiments, the at least one spot weld feature comprises a single spot weld feature positioned between the second meltable feature and an edge of the surface.

In some embodiments, the first meltable material, the second meltable material and the third meltable material are a same meltable material.

While exemplary embodiments have been described herein, it should be appreciated that various changes in form and detail can be made without departing from the intended scope of the claims appended hereto.

Although the present invention is defined in the attached claims, it should be understood that the present invention can also (alternatively) be defined in accordance with the following embodiments:
1. An on-body medical device, comprising:
   a surface;
   an adhesive layer for securing the medical device to skin of a user, the adhesive layer containing a substrate on which an adhesive is applied; and
   meltable thermal weld features formed on the surface for securing the adhesive layer to the surface when melted, the meltable thermal weld features comprising:
      a first meltable structure of a substantially uniform amount of a first meltable material above a melting border along a length of the first structure, wherein a portion of the first meltable material at a height above the melting border is configured to melt, and
      a second meltable structure, where the second meltable structure is configured to have a gradient in amount of a second meltable material above a second melting border that extends along at least a portion of a length of the second meltable structure that is configured to melt.
2. The on-body medical device of embodiment 1, wherein the first meltable structure is of a substantially uniform height above the melting border.
3. The on-body medical device of embodiment 2, wherein the second meltable structure comprises a gradient structure of the second meltable material that decreases in height above the second melting border along at least a portion of the gradient structure.
4. The on-body medical device of embodiment 3, further comprising an additional gradient structure of the second meltable material that decreases in height above a third melting border along at least a portion of the additional gradient structure.
5. The on-body medical device of embodiment 1, wherein the surface has a width, wherein the first meltable structure extends across a central portion of the width of the surface, and wherein the second meltable structure extends across a portion of the surface between the central portion of the width and an edge of the surface.
6. The on-body medical device of embodiment 5, wherein the on-body medical device further comprises an additional meltable structure configured to have a gradient in amount of meltable material above a third melting border along at least a portion of its length and wherein the second meltable structure and the additional meltable structure are formed on opposite ends of the first meltable structure along the width of the surface.
7. The on-body medical device of embodiment 6, wherein the second meltable structure and the additional meltable structure are arcuate raised ribs of the meltable material.
8. The on-body medical device of embodiment 1, wherein the first meltable structure comprises a raised rib of the meltable material.
9. The on-body medical device of embodiment 1, wherein the first meltable material is a same meltable material as the second meltable material.
10. An on-body medical device, comprising:
   a surface;
   an adhesive layer for securing the medical device to skin of a user, the adhesive layer containing a substrate on which an adhesive is applied;
   meltable thermal weld features formed on the surface for securing the adhesive layer to the surface when melted, the meltable thermal weld feature comprising:
      a first meltable structure of a first meltable material having a substantially uniform amount of the first meltable material above a melting border along a length of the first meltable structure, wherein first meltable material at a height above the melting border is configured to melt,
      a second meltable structure of a second meltable material, wherein the second meltable structure is configured to have a gradient in amount of the second meltable material above a second melting border that extends along at least a portion of a length of the second meltable structure, and
      at least one spot weld feature of a third meltable material formed on the surface positioned and configured to be weakest of the weld features once melted to form a thermal weld.
11. The on-body medical device of embodiment 10, wherein the at least one spot weld feature comprises multiple spot weld features.
12. The on-body medical device of embodiment 11, wherein the at least one spot weld feature comprises a single spot weld feature positioned between the second meltable feature and an edge of the surface.
13. The on-body medical device of embodiment 10, wherein the first meltable material, the second meltable material and the third meltable material are a same meltable material.
14. An on-body medical device, comprising:
   a surface;
   an adhesive layer for securing the medical device to skin of a user, the adhesive layer containing a substrate on which an adhesive is applied;
   a main thermal weld structure containing meltable material for securing the adhesive layer to the surface; and
   dot thermal weld structures containing the meltable material for additional securing of the adhesive layer to the surface.
15. The on-body medical device of embodiment 14, wherein the dot thermal weld structures include dot thermal weld structures of different diameters.
16. The on-body medical device of embodiment 14, wherein the dot thermal weld structures include dot thermal weld structures of different heights.
17. The on-body medical device of embodiment 14, wherein the dot thermal weld structures are grouped into spatially segregated groups on the housing.
18. The on-body medical device of embodiment 17, wherein at least one of the spatially segregated groups contains thermal weld structures of different heights.
19. The on-body medical device of embodiment 17, wherein at least one of the spatially segregated groups contains thermal weld structures of different diameters.
20. The on-body medical device of embodiment 17, wherein average spacing between dot thermal weld structures of a first of the spatially segregated groups is at least 20 percent less than the average spacing between dot weld thermal structures of a second of the spatially segregated group.
21. An on-body medical device, comprising:
   a surface;
   an adhesive layer for securing the medical device to skin of a user, the adhesive layer containing a substrate on which an adhesive is applied; and
   thermal welds formed on the surface for securing the adhesive layer to the surface, the thermal welds comprising:
      a first weld structure of a substantially uniform amount of a first meltable material along a length of the first structure, and
      a second weld structure, where the second weld structure has a gradient in amount of a second meltable material that extends along at least a portion of a length of the second weld structure.
22. The on-body medical device of embodiment 21, wherein the first weld structure is of a substantially uniform thickness above the melting border.
23. The on-body medical device of embodiment 21 or 22, wherein the second weld structure comprises a gradient structure of the second meltable material that decreases in thickness along at least a portion of the gradient structure.
24. The on-body medical device of any one of embodiments 21 to 23, further comprising an additional gradient weld structure of the second meltable material that decreases in thickness along at least a portion of the additional gradient structure.
25. The on-body medical device of any one of embodiments 21 to 24, wherein the surface has a width, wherein the first weld structure extends across a central portion of the width of the surface, and wherein the second weld structure extends across a portion of the surface between the central portion of the width and an edge of the surface.
26. The on-body medical device of any one of embodiments 21 to 25, wherein the on-body medical device further comprises an additional weld structure having a gradient in amount of meltable material along at least a portion of its length and wherein the second weld structure and the additional weld structure are formed on opposite ends of the first weld structure along the width of the surface.
27. The on-body medical device of embodiment 26, wherein the second weld structure and the additional weld structure are arcuate raised ribs of the meltable material.
28. The on-body medical device of any one of embodiments 21 to 27, wherein the first weld structure comprises a raised rib of the meltable material.
29. The on-body medical device of any one of embodiments 21 to 28, wherein the first meltable material is a same meltable material as the second meltable material.
30. An on-body medical device, comprising:
   a surface;
   an adhesive layer for securing the medical device to skin of a user, the adhesive layer containing a substrate on which an adhesive is applied;
   thermal welds formed on the surface for securing the adhesive layer to the surface, the thermal welds comprising:
      a first weld structure of a first meltable material having a substantially uniform amount of the first meltable material above along a length of the first meltable structure,
      a second weld structure of a second meltable material, wherein the second meltable structure is configured to have a gradient in amount of the second meltable material above that extends along at least a portion of a length of the second meltable structure, and
      at least one spot weld feature of a third meltable material formed on the surface positioned and configured to be weakest of the thermal welds.
31. The on-body medical device of embodiment 30, wherein the at least one spot weld feature comprises multiple spot weld features.
32. The on-body medical device of embodiment 30 or 31, wherein the at least one spot weld feature comprises a single spot weld feature positioned between the second meltable feature and an edge of the surface.
33. The on-body medical device of any one of embodiment 30 to 32, wherein the first meltable material, the second meltable material and the third meltable material are a same meltable material.

## Claims

1. An on-body medical device, comprising:
a surface;
an adhesive layer for securing the medical device to skin of a user, the adhesive layer containing a substrate on which an adhesive is applied; and
meltable thermal weld features formed on the surface for securing the adhesive layer to the surface when melted, the meltable thermal weld features comprising:
a first meltable structure of a substantially uniform amount of a first meltable material above a melting border along a length of the first structure, wherein a portion of the first meltable material at a height above the melting border is configured to melt, and
a second meltable structure, where the second meltable structure is configured to have a gradient in amount of a second meltable material above a second melting border that extends along at least a portion of a length of the second meltable structure that is configured to melt.

2. The on-body medical device of claim 1, wherein the first meltable structure is of a substantially uniform height above the melting border.

3. The on-body medical device of claim 1 or 2, wherein the second meltable structure comprises a gradient structure of the second meltable material that decreases in height above the second melting border along at least a portion of the gradient structure.

4. The on-body medical device of any one of claims 1 to 3, further comprising an additional gradient structure of the second meltable material that decreases in height above a third melting border along at least a portion of the additional gradient structure.

5. The on-body medical device of claim 1 to 4, wherein the surface has a width, wherein the first meltable structure extends across a central portion of the width of the surface, and wherein the second meltable structure extends across a portion of the surface between the central portion of the width and an edge of the surface.

6. The on-body medical device of any one of claims 1 to 5, wherein the on-body medical device further comprises an additional meltable structure configured to have a gradient in amount of meltable material above a third melting border along at least a portion of its length and wherein the second meltable structure and the additional meltable structure are formed on opposite ends of the first meltable structure along the width of the surface.

7. The on-body medical device of claims 5 or 6, wherein the second meltable structure and the additional meltable structure are arcuate raised ribs of the meltable material and/or wherein the first meltable structure comprises a raised rib of the meltable material.

8. The on-body medical device of any preceding claim, wherein the on-body medical device comprises at least one spot weld feature of a third meltable material formed on the surface positioned and configured to be weakest of the weld features once melted to form a thermal weld.

9. The on-body medical device of claim 8, wherein the at least one spot weld feature comprises multiple spot weld features, or wherein the at least one spot weld feature comprises a single spot weld feature positioned between the second meltable feature and an edge of the surface.

10. The on-body medical device of any preceding claim, wherein the first meltable material, the second meltable material and/or the third meltable material are a same meltable material.

11. An on-body medical device, comprising:
a surface;
an adhesive layer for securing the medical device to skin of a user, the adhesive layer containing a substrate on which an adhesive is applied;
a main thermal weld structure containing meltable material for securing the adhesive layer to the surface; and
dot thermal weld structures containing the meltable material for additional securing of the adhesive layer to the surface.

12. The on-body medical device of claim 11, wherein the dot thermal weld structures include dot thermal weld structures of different diameters and/or different heights.

13. The on-body medical device of claim 14, wherein the dot thermal weld structures are grouped into spatially segregated groups on the housing, in particular wherein at least one of the spatially segregated groups contains thermal weld structures of different heights and/or diameters.

14. An on-body medical device, comprising:
a surface;
an adhesive layer for securing the medical device to skin of a user, the adhesive layer containing a substrate on which an adhesive is applied; and
thermal welds formed on the surface for securing the adhesive layer to the surface, the thermal welds comprising:
a first weld structure of a substantially uniform amount of a first meltable material along a length of the first structure, and
a second weld structure, where the second weld structure has a gradient in amount of a second meltable material that extends along at least a portion of a length of the second weld structure.

15. An on-body medical device, comprising:
a surface;
an adhesive layer for securing the medical device to skin of a user, the adhesive layer containing a substrate on which an adhesive is applied;
thermal welds formed on the surface for securing the adhesive layer to the surface, the thermal welds comprising:
a first weld structure of a first meltable material having a substantially uniform amount of the first meltable material above along a length of the first meltable structure,
a second weld structure of a second meltable material, wherein the second meltable structure is configured to have a gradient in amount of the second meltable material above that extends along at least a portion of a length of the second meltable structure, and
at least one spot weld feature of a third meltable material formed on the surface positioned and configured to be weakest of the thermal welds.
